# EUROPEAN PATENT APPLICATION

(11) **EP 1 878 342 A1**
(43) Date of publication of application: **16.01.2008**
(21) Application number: 06291151.6
(22) Date of filing: 13.07.2006
(51) Int. Cl.: A01K 67/027, C12N 15/85

(54) **Immunodeficient mice transgenic for HLA class I and HLA class II molecules and their uses**

(71) Applicant: INSTITUT PASTEUR, 75015 Paris (FR)
(72) Inventor: Garcia, Sylvie, 94230 Cachan (FR); Stockinger, Brigitta, NW7 4ASD London (GB)
(74) Representative: Goulard, Sophie

(57) **Abstract**

The invention relates to mice which are genetically deprived of T, B lymphocytes and NK cells, deficient for murine MHC class I and/or MHC class II molecules, and transgenic for the expression of the HLA class I and/or HLA class II molecules, and to their use as recipient hosts for the transplantation of human haematopoietic precursors, to study the human adaptative immune system development and function *in vivo.* The invention relates also to the applications of this human/mouse chimera model to improve immunotherapy against pathogens, cancer and autoimmune diseases.

## Description

The invention relates to mice which are genetically deprived of T, B lymphocytes and NK cells, deficient for murine MHC class I and/or MHC class II molecules, and transgenic for the expression of the HLA class I and/or HLA class II molecules. The invention relates also to the use of the transgenic mice of the invention as recipient hosts for the transplantation of human haematopoietic precursors, to study the human adaptative immune system development and function *in vivo.* The invention relates also to the applications of this human/mouse chimera model to improve immunotherapy against pathogens, cancer and autoimmune diseases.

In order to analyze human immune system development and function *in vivo,* a number of studies have been trying to reproduce human hematopoiesis in small animal xenotransplantation models. In these models, human hematopoietic cells and tissues are transplanted into mice that are compromised in their capacity to reject xenografts, so as to generate human/mouse chimera or humanized mice.

Engraftment was first reported after transfer of mature human peripheral blood leukocytes in severe combined immunodeficient mice (huPBL-SCID mice; Mosier et al., Nature, 1998, 335, 256-259) and transplantation of blood-forming fetal liver cells, fetal bone, fetal thymus and fetal lymph nodes in SCID mice (SCID-hu mice; McCune et al., Science, 1988, 241, 1632-1639; McCune et al., Semin. Immunol., 1996, 8, 187-). After these initial reports in T and B lymphocyte-deficient SCID mice (Prkdc^{scid} mutant mice), some level of engraftment was also achieved by transplantation of blood-forming cells in recombination activating gene (RAG)-deficient mice (Rag1^{-/-}, Rag2^{-/-} mutant mice; Schultz et al., J. Immunol., 2000, 164, 2496-2507; Goldman et al., Br. J. Haematol., 1998, 103, 335-342). The engraftment levels in these models, however, were still low presumably due to the remaining innate immunity of host animals. Nonobese diabetic/severe combined immunodeficient (NOD/SCID) mice have been shown to support higher levels of human progenitor cells engraftment than BALB/c/SCID or C.B.17/SCID mice (Greiner *et al.,* Stem Cells, 1998, 16, 166-). NOD/SCID mice harboring either a null allele at the beta-2 microglobulin gene (NOD/SCID/β2m^{-/-}; O. Kollet et al., Blood, 2000, 95, 3102-) or a truncated common cytokine receptor γ chain (γc) mutant lacking its cytoplasmic region (NOD/SCID/γ_{c}^{-/-}; Ito et al., Blood, 2002, 100, 3175-) were developed. In these mice, NK-as well as T- and B-cell development and functions are disrupted, because β2m is necessary for major histocompatibility complex (MHC) class 1-mediated innate immunity, and because γc (originally called IL-2Rγ chain) is an indispensable component of receptor heterodimers for many lymphoid-related cytokines (IL-2, IL-7, IL-9, IL-12, IL-15 and IL-21), whose some are required for generation/maintenance of lymphoid lineages. However, these models sustain only limited development and maintenance of human lymphoid cells and rarely produce immune responses.

More recently, intrahepatic injection of CD34⁺ human cord blood cells into irradiated newborn Rag2^{-/-}/γ_{c}^{-/-} mice led to the formation of a quantitatively and functionally complete immune system, as demonstrated by *de novo* development of B, T, and dendritic cells; formation of structured primary and secondary lymphoid organs; and production of functional immune response (Traggai et al., Science, 2004, 304, 104-107). The efficiency of this xenogenic transplantation system was further confirmed in the same mouse strain (Gimeno et al., Blood, 2004, 104, 3886-3883) as well as in NOD/SCID mice harboring a complete null mutation of the common cytokine receptor γ chain (NOD/SCID/γ_{c}^{null}; Ishikawa et al., Blood, 2005, 106, 1565-1573).

Nevertheless, this model presents several disadvantages: the quantitative T cell reconstitution is very poor, with a very limited number of T cells in the chimera lymphoid organs. Furthermore, functional studies are difficult in this system since the nature of the antigen presenting cells (murine *versus* human) remains unclear and do not allow to exclude the possible restriction of human T cell responses by murine MHC.

To overcome these limitations, the present invention provides a transgenic mouse, characterized in that it has a phenotype comprising:
a) a deficiency for murine T lymphocytes, B lymphocytes and NK cells,
b) a deficiency for murine MHC class I and MHC class II molecules,
c) a functional xenogenic MHC class I transgene and/or a functional xenogenic MHC class II transgene.

The xenogenic MHC class I and/or MHC class II functional transgene(s) may be human HLA class I and/or HLA class II functional transgene(s). Alternatively, they may be simian functional MHC class I and/or MHC class II transgene(s) such as Patr-class I and/or -class II chimpanzee genes or Mamu-class I or -class II macaque genes. The simian MHC genes are useful to understand some differences for AIDS susceptibility (Bontrop, R. E.; and D. I. Watkins, Trends in Immunol., 2005, 26: 227-233).

The substitution of the host murine MHC molecules by HLA molecules, in the transgenic mice according to the present invention, improves considerably the initial models. In a HLA context, the human CD4/MHC class II and CD8/MHC class I interactions which are stronger than the xenogenic interactions mainly encountered in the previous models increase drastically the T cell number both by facilitating positive selection of human thymocytes and peripheral survival of generated human T cells. In addition, the substitution of the host murine MHC molecules by HLA molecules ensures the restriction of human T cell response in a human MHC context.

Mice of the invention are used as recipient hosts for human haematopoietic precursors transplantation, to generate new human/mouse chimera. These mice represent a completely humanized model, easy to set up, that can be used to study the development of the human immune adaptative system, *in vivo.* These mice provide a model useful in the development and optimization of vaccines or immunotherapies with maximum efficacy *in vivo* for human use. Specifically, such mice enable a complete analysis of the components of the immune adaptative response (antibody, helper and cytolytic) to a human antigen (pathogen, tumor, auto-antigen) in a single animal, as well as an evaluation of the protection specifically conferred by vaccination against an antigenic challenge. In this model it is possible to detect the presence of antigen-specific antibodies, antigen specific HLA class II restricted CD4⁺ T cell response, and antigen specific HLA class I restricted CD8⁺ T cell response. It is also possible to study how these responses cooperate. Therefore, this model is useful to set up more efficient prophylactic or curative immunotherapies against human pathogens, cancer and auto-immune diseases. These mice represent an optimized tool for basic and applied immunology studies.

### Definitions

- "deficiency for" refers to the lack of a molecular or cellular function.
- "mutation"refers to the substitution, insertion, deletion of one or more nucleotides in a polynucleotide sequence.
- "deficient gene", "inactivated gene", "null allele" refers to a gene comprising a spontaneous or targeted mutation that results in an altered gene product lacking the molecular function of the wild-type gene.
- "disrupted gene" refers to a gene that has been inactivated using homologous recombination or other approaches known in the art.
- "transgene" refers to a nucleic acid sequence, which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can be operably linked to one or more transcriptional regulatory sequences and any other nucleic acid, such as introns, that may be necessary for optimal expression of a selected nucleic acid.
- "functional transgene" refers to a transgene that produces an mRNA transcript, which in turn produces a properly processed protein in at least one cell of the mouse comprising the transgene. One of skill will realize that the diverse set of known transcriptional regulatory elements and sequences directing post-transcriptional processing provide a library of options from which to direct the expression of a transgene is a host mouse. In many embodiments of the invention, expression of an HLA transgene under the control of an H-2 gene regulatory element may be preferred.
- "HLA" refers to the human MHC complex and "H-2" to the mouse MHC complex.
- "xenogenic", "xenogenic species" refers to a non-mouse vertebrate.

The transgenic mouse according to the present invention which is deficient for murine T and B lymphocytes, and NK cells, comprises two genes essential in T, B and/or NK cells development that are inactivated by a spontaneous mutation or a targeted mutation (deficient genes). These mutations which are well-known to those of ordinary skill in the art include, for example: a first mutation which is the mouse *scid* mutation (Prkdc^{scid}; Bosma et al., Nature 1983, 301, 527-530; Bosma et al., Curr. Top. Microbiol., Immunol., 1988, 137, 197-202) or the disruption of the recombination activating gene (Rag1^{-/-} or Rag2^{-/-}; Mombaerts et al., Cell, 1992, 68, 869-877; Takeda et al., Immunity, 1996, 5, 217-228), and a second mutation which is the beige mutation (Lyst^{bg}; Mac Dougall et al., Cell. Immunol., 1990, 130, 106-117) or the disruption of the β₂-microglobulin gene (β2m^{-/-}; Kollet et al., Blood, 2000, 95, 3102-3105), the IL-2 receptor γ chain (or common cytokine receptor γ chain (γ_{c})) gene (IL-2Rγ^{-/-} or γ_{c}^{-/-}; DiSanto et al., P.N.A.S., 1995, 92, 377-381), or the IL-2 receptor β chain (IL-2Rβ) gene (IL-2Rβ^{-/-}; Suzuki et al; J. Exp. Med., 1997, 185, 499-505)

In addition these mutations are in an appropriate genetic background which is well-known to those of ordinary skill in the art. For example, the SCID mutation is preferably in a NOD background (diabetes-susceptible Non-obese Diabetic back-ground, NOD/SCID; Prochazka et al., P.N.A.S., 1992, 89, 3290-3294).

For example, the mouse according to the present invention comprises one of the following genotypes corresponding to a T, B and NK cell deficiency : SCID/Beige (*scid*/*scid, bg*/*bg ;* Mac Dougall et al., Cell. Immunol., 1990, 130, 106-117), NOD/SCID/IL2-Rγ^{null} (Ishikawa et al., Blood, 106, 1565-1573; Schultz et al., J. Immunol., 2005, 174, 6477-6489), NOD/SCID/β₂m^{-/-} (Zijlstar et al., Nature, 1990, 344, 742-746), Rag2^{-/-}/γ_{c}^{-/-} (Goldman et al., Br. J. Haematol., 1998, 103, 335-342).

In a first embodiment, the invention provides a transgenic mouse wherein the deficiency in a) is associated with a deficient Rag2 gene and a deficient common receptor γ chain gene. Preferably, both genes are disrupted by homologous recombination (Rag2 and γ_{c} knock-out or Rag2^{-/-}/γ_{c}^{-/-}).

The MHC class I molecule comprises an α-chain (heavy chain) which is non-covalently associated with a β2-microglobulin (β2-m) light chain. The MHC class II molecules are heterodimers comprising an α-chain and a β-chain. The human complex comprises three class I α-chain genes: HLA-A, HLA-B, and HLA-C and three pairs of MHC class IIα- and β- chain genes, HLA-DR, -DP, and -DQ. In many haplotypes, the HLA-DR cluster contains an extra β-chain gene whose product can pair with the DRα chain, and so the three sets of genes give rise to four types of MHC class II molecules. In the mouse, the three class I α-chain genes are H-2-L, H-2-D, and H-2K. The mouse MHC class II genes are H-2-A and H-2-E. H-2-Eα is a pseudogene in the H2^{b} haplotype.

In a second embodiment, the invention provides a transgenic mouse wherein the deficiency in murine MHC class I molecules is associated with a deficient β2-microglobulin gene, preferably a disrupted β2-microglobulin gene (β₂m knock-out, β₂m^{-/-}); the absence of β2-microglobulin chain in the mouse leads to lack of murine MHC class I molecules (H-2-L, H-2-D, and H-2K) cell surface expression.

In a preferred embodiment, the β2-microglobulin gene and at least one of the class I α-chain genes, for example the H2-D^{b} and/or H2-K^{b} genes, are disrupted.

β₂m knock-out mice are well-known to those of ordinary skill in the art; for example, β₂m^{-/-} mice are described in Zijlstar et al., Nature, 1990, 344, 742-746, and β₂m^{-/-}, H2-D^{b-/-} and/or H2-K^{b-/-} mice can be obtained as described in Pascolo et al., J. Exp. Med., 1997, 185:2043-2051.

In a third embodiment, the invention provides a transgenic mouse wherein the deficiency in murine MHC class II molecules is associated with a deficient H-2-Aβ gene, and eventually a deficient H-2-Eβ gene.

In a H2^{b} haplotype, the deficiency in murine MHC class II molecules is obtained by disrupting the H-2^{b}-Aβ gene, only (I-Aβ^{b} knock-out or I-Aβ^{b-/-}); the absence of H-2 I-A β-chain leads to lack of conventional H-2 I-A and I-E class II molecules cell surface expression, since H-2-Eα is a pseudogene in this haplotype. In the other H-2 haplotypes, the deficiency in murine MHC class II molecules is obtained by disrupting both H-2-Aβ and H-2-Eβ genes.

I-Aβ^{b} knock-out mice are well-known to those of ordinary skill in the art. For example, I-Aβ^{b -/- mice} are described in Takeda *et al.,* Immunity, 1996, 5, 217-228. Mice knocked out for both H-2-Aβ gene and H-2-Eβ gene are described in Madsen et al., Proc. Natl. Acad. Sci. USA, 1999, 96:10338-10343.

One of the difficulties hampering the design of T-epitope-based vaccines targeting T lymphocytes is HLA class I/class II molecule polymorphism. It is known in the art that genetic diversity exists between the HLA genes of different individuals as a result of both polymorphic HLA antigens and distinct HLA alleles Due to the high degree of polymorphism of the HLA molecules, the set of epitopes from an antigen, which are presented by two individuals may be different depending on the HLA molecules or HLA type which characterize said individuals. However, despite of a high number of HLA molecules whose repartition is not homogeneous worldwide, some alleles are predominant in human populations (HLA-DR1, -DR3, - B7, -B8, -A1, -A2). For example, HLA-A2.1 and HLA-DR1 molecules are expressed, by 30 to 50 % and 6 to 18 % of individuals, respectively. In addition, there is a redundancy of the presented set of peptides between HLA class I isotypic or allelic variants and the binding of peptides to HLA class II molecules is less restrictive than to class I molecules. Therefore, the peptides which are presented by the HLA-A2.1 and HLA-DR1 molecules should be representative of the epitopes that are presented by most individuals of the population. Nethertheless, it may be desirable to identify the optimal epitopes that are presented by other HLA isotypic or allelic variants, to cover the overall human population. This may be also important in cases where there is a bias in the immune response, so that the antigen is preferably presented by other HLA haplotypes. This may be also relevant in cases where the HLA haplotypes are involved in disease development or outcome, for example auto-immune diseases (Jones et al., Nature Reviews Immunol., 2006, 6:271-282) or viral infections like HCV (LJ Yee, Genes and Immunity, 2004, 5:237-245)or HIV (Bontrop R.E. and Watkins D.I., Trends in Immunol., 2005, 26:227-233).

The transgenic mouse according to the present invention may comprise one or more functional xenogenic MHC class I and/or class II transgene(s). Preferably the allotypes of the MHC class I and/or class II transgenes are reflective of the genetic variability of the xenogenic population. For example, the allotypes which are the most frequent in the xenogenic population are chosen so as to cover the overall xenogenic population.

In a fourth embodiment, the invention provides a transgenic mouse wherein the xenogenic MHC class I transgene is a human HLA class I transgene and the xenogenic MHC class II transgene is a human HLA class II transgene.

Preferably, the human HLA class I and class II transgenes correspond to allotypes which are the most frequent in the human population.

For example, the HLA class I transgene is an HLA-A2 transgene and the HLA class II transgene is an HLA-DR1 transgene. More preferably, the HLA-A2 transgene encodes a HLA-A2.1 monochain in which the human β2m is covalently linked by a peptidic arm to the HLA-A2.1 heavy chain. The HLA-DR1 α and β chains may be encoded by the HLA-DRA*0101 and the HLA-DRB1*0101 genes, respectively.

HLA class I and HLA class II transgenic mice are well-known to those of ordinary skill in the art. For example, HLA-A2.1 transgenic mice expressing a chimeric monochain (α1-α2 domains of HLA-A2.1 (encoded by HLA-A*0201 gene), α3 to cytoplasmic domains of H-2 D^{b}, linked at its N-terminus to the C terminus of human β2m by a 15 amino-acid peptide linker) are described in Pascolo et al., J. Exp. Med., 1997, 185, 2043-2051. HLA-DR1 transgenic mice expressing the DR1 molecule encoded by the HLA-DRA*0101 and HLA-DRB1*0101 genes are described in Altmann et al., J. Exp. Med., 1995, 181, 867-875.

Accordingly, embodiments of the invention disclosed herein may substitute one polymorphic HLA antigen for another or one HLA allele for another. Examples of HLA polymorphisms and alleles can be found, for example, at http://www.anthonynolan.org.uk/HIG/data.html and http://www.ebi.ac.uk/imgt/hla. and in Genetic diversity of HLA: Functional and Medical Implication, Dominique Charon (Ed.), EDK Medical and Scientific International Publisher, and The HLA FactsBook, Steven G.E. Marsh, Peter Parham and Linda Barber, AP Academic Press, 2000.

The human HLA class I and class II transgenes may also correspond to allotypes that are involved in disease development or outcome, for example auto-immune diseases (Jones et al., Nature Reviews Immunol., 2006, 6:271-282) or viral infections like HCV (Yee L.J., Genes and Immunity, 2004, 5:237-245)or HIV (Bontrop R.E. and D.I.Watkins, Trends in Immunol., 2005, 26:227-233).

Alternatively, MHC class I and class II molecules from other vertebrates can be expressed in the transgenic mice according to the present invention. MHC gene sequences are accessible in the data bases such as the NCBI database (http://www.ncbi.nlm.nih.gov/).

In a fifth embodiment, the invention provides a transgenic mouse having one of the following genotypes:
- Rag2^{-/-}, γ_{c}^{-/-}, β₂m^{-/-}, I-Aβ^{b-/-}, HLA-A2^{+/+}, HLA-DR1^{+/+},
- Rag2^{-/-}, γ_{c}^{-/-}, β₂m^{-/-}, I-Aβ^{b-/-}, HLA-A2^{+/+}, and
⁻ Rag2^{-/-}, γ_{c}^{-/-}, β₂m^{-/-}, I-Aβ^{b-/-}, HLA-DR1^{+/+}.

In a sixth embodiment, the invention provides a transgenic mouse, further comprising a deficiency for the C5 protein of complement (C5^{-/-}). The mouse of the strains 129 or FBV are examples of mice having a deficiency for the C5 protein of complement. These strains can advantageously be used for making the transgenic mouse according to the present invention.

The mouse of the present invention may be produced by successive crossing of mice carrying one or more mutation(s)/transgene(s) of interest as defined above, and screening of the progenies until double mutants and double transgenics are obtained.

They are advantageously produced by crossing a H-2 class I-/class II-KO immunodeficient mice, with a HLA-I⁺ and/or a HLA-II⁺ transgenic mouse as defined above.

Therefore, the present invention relates also to a transgenic mouse, characterized in that it has a phenotype comprising :
a) a deficiency for murine T and B lymphocytes, and NK cells, and
b) a deficiency for murine MHC class I and MHC class II molecules.

Preferably, said H-2 class I-/class II- KO immunodeficient mice has a Rag2^{-/-}, γ_{c}^{-/-}, β₂m^{-/-}, I-Aβ^{b-/-} genotype.

Based on the disclosure herein, additional MHC class I/MHC class II-transgenic, H-2 class I/class II-KO immunodeficient mice can be constructed by using conventional homologous recombination techniques. For example, additional MHC class I transgenic and additional MHC class II-transgenic may be constructed using conventional homologous recombination techniques. These transgenics may be crossed with H-2 class I-/class II-KO immunodeficient mice as defined above.

"Homologous recombination" is a general approach for targeting mutations to a preselected, desired gene sequence of a cell in order to produce a transgenic animal (Mansour et al., Nature, 1988, 336:348-352 ; Capecchi, M. R., Trends Genet., 1989, 5:70-76; Capecchi, M. R., Science, 1989, 244:1288-1292 ; Capecchi et al., In: Current Communications in Molecular Biology, Capecchi, M R. (Ed.), Cold Spring Harbor Press, Cold Spring Harbor, N. Y (1989), pp. 45-52*;* Frohman et al., Cell, 1989, 56:145-147). It is now feasible to deliberately alter any gene in a mouse (Capecchi, M. R., Trends Genet., 1989, 5:70-76 ; Frohman et al., Cell, 1989, 56:145-147). Gene targeting involves the use of standard recombinant DNA techniques to introduce a desired mutation into a cloned DNA sequence of a chosen locus. In order to utilize the "gene targeting" method, the gene of interest must have been previously cloned, and the intron-exon boundaries determined. The method results in the insertion of a marker gene (e.g., an nptll gene) into a translated region of a particular gene of interest. Thus, use of the gene targeting method results in the gross destruction of the gene of interest. Significantly, the use of gene targeting to alter a gene of a cell results in the formation of a gross alteration in the sequence of that gene. That mutation is then transferred through homologous recombination to the genome of a pluripotent, embryo-derived stem (ES) cell. The altered stem cells are microinjected into mouse blastocysts and are incorporated into the developing mouse embryo to ultimately develop into chimeric animals. In some cases, germ line cells of the chimeric animals will be derived from the genetically altered ES cells, and the mutant genotypes can be transmitted through breeding.

The chimeric or transgenic animal cells of the present invention may be prepared by introducing one or more DNA molecules into a cell, which may be a precursor pluripotent cell, such as an ES cell, or equivalent (Robertson, E. J., In: Current Communications in Molecular Biology, Capecchi, M. R. (ed.), Cold Spring Harbor Press, Cold Spring Harbor, N.Y. (1989), pp. 39-44)*.* The term "precursor" is intended to denote only that the pluripotent cell is a precursor to the desired ("transfected") pluripotent cell, which is prepared in accordance with the teachings of the present invention. The pluripotent (precursor or transfected) cell can be cultured *in vivo* in a manner known in the art (Evans et al., Nature, 1981, 292:154-156), to form a chimeric or transgenic animal. Any ES cell can be used in accordance with the present invention. It is, however, preferred to use primary isolates of ES cells. Such isolates can be obtained directly from embryos, such as the CCE cell line disclosed by Robertson, E. J. (In: Current Communications in Molecular Biology, Capecchi, M R. (ed.), Cold Spring Harbor Press, Cold Spring Harbor, N.Y. (1989), pp. 39-44*),* or from the clonal isolation of ES cells from the CCE cell line (Schwartzberg et al., Science, 1989, 246:799-803, which reference is incorporated herein by reference). Such clonal isolation can be accomplished according to the method of E. J. Robertson *(In: Teratocarcinomas and Embryonic Stem Cells: A Practical Approach, (E. J. Robertson, Ed), IRL Press, Oxford, 1987),* which reference and method are incorporated herein by reference. The purpose of such clonal propagation is to obtain ES cells, which have a greater efficiency for differentiating into an animal. Clonally selected ES cells are approximately 10-fold more effective in producing transgenic animals than the progenitor cell line CCE. For the purposes of the recombination methods of the present invention, clonal selection provides no advantage. An example of ES cell lines which have been clonally derived from embryos, are the ES cell lines, ABI (hprt⁺) or AB2.1 (hprt"). The ES cells are preferably cultured on stromal cells (such as STO cells (especially SNC4 STO cells) and/or primary embryonic fibroblast cells) as described by E. J. Robertson (In: Teratocarcinomas and Embryonic Stem Cells: A Practical Approach, (E. J Robertson, Ed., IRL Press, Oxford, 1987, pp 71-112), which reference is incorporated herein by reference. Methods for the production and analysis of chimeric mice are disclosed by Bradley, A. (In: Teratocarcinomas and Embryonic Stem Cells: A Practical Approach, (E. J Robertson, Ed.), IRL Press, Oxford, 1987, pp 113-151)*,* which reference is incorporated herein by reference. The stromal (and/or fibroblast) cells serve to eliminate the clonal overgrowth of abnormal ES cells. Most preferably, the cells are cultured in the presence of leukocyte inhibitory factor ("lit") (Gough et al., Reprod. Fertil. Dev., 1989, 1:281-288; Yamamori et al., Science, 1989, 246:1412-1416). Since the gene encoding lif has been cloned (Gough et al., Reprod. Fertil. Dev., 1989, 1:281-288), it is especially preferred to transform stromal cells with this gene, by means known in the art, and to then culture the ES cells on transformed stromal cells that secrete lif into the culture medium.

The invention relates also to an isolated cell from a transgenic mouse as defined above. Preferably, said cell has one of the following genotypes:
- Rag2^{-/-}, γ_{c}^{-/-}, β₂m^{-/-}, I-Aβ^{b-/-}, HLA-A2^{+/+}, HLA-DR1^{+/+},
- Rag2^{-/-}, γ_{c}^{-/-}, β₂m^{-/-}, I-Aβ^{b-/-}, HLA-A2^{+/+},
⁻ Rag2^{-/-}, γ_{c}^{-/-}, β₂m^{-/-}, I-Aβ^{b-/-}, HLA-DR1^{+/+}, and
- Rag2^{-/-}, γ_{c}^{-/-}, β₂m^{-/-}, I-Aβ^{b-/-}.

The invention relates also to the use of the transgenic mouse as defined above as a recipient host for the transplantation of xenogenic hematopoietic precursors from the same species as the MHC class I and/or class II transgene(s), for example human hematopoietic precursors.

The invention relates also to a method for producing a chimeric mouse having a functional xenogenic immune system, comprising the step of transplanting xenogenic hematopoietic precursors from the same species as the MHC class I and/or class II transgene(s), for example human hematopoietic progenitor cells, into a transgenic mouse as defined above.

The MHC haplotype of the murine host should not be obligatory the one of the donor cells to ensure the previous functions. Therefore, the xenogenic MHC class I and/or class II transgenes may have the sequence of the alleles that are present in the precursor cells or the sequence of other alleles that are not present in the precursor cells. If the MHC haplotype is different between the murine hosts and the donor cells, the donor T cells will be educated in both host and its MHC context.

The hematopoietic progenitor cells, for example the human hematopoietic precursors, and the methods of transplanting such cells into mice are well-known in the art (Traggiai et al., Science, 2004, 304, 104-107; Ishikawa et al., Blood, 2005, 106, 1565-1573; Gimeno et al., Blood, 2004, 104, 3886-3893). Human progenitors can be issued from different sources such as cord blood, fetal liver, adult or embryonic stem cells. For example, human CD34+ cells (from 10⁴ to 10⁶ cells), isolated from cord blood or fetal liver are transplanted intraperitoneally, intra-hepatically, or via a facial vein, into sub-lethally irradiated newborn transgenic mice.

The hematopoietic precursors are preferably from donors of different MHC haplotypes, more preferably of the haplotypes that are the most frequent in the xenogenic species, to take into account the xenogenic MHC polymorphism. For example, the mice are transgenic for the HLA haplotypes that are the most frequent in the human population. These humanized mice have HLA molecules that are reflective of the genetic variability of the human population. Therefore, their immune system is reflective of the immune system of most individuals of the population.

The haplotype of the precursor cells may also correspond to an haplotype that is involved in disease development or outcome, for example auto-immune diseases (Jones et al., Nature Reviews Immunol., 2006, 6:271-282) or viral infections like HCV (Yee L.J., Genes and Immunity, 2004, 5:237-245)or HIV (Bontrop R.E. and D.I.Watkins, Trends in Immunol., 2005, 26:227-233).

The invention relates also to the use of the chimeric mouse as defined above as a model to study the development of the adaptative immune system of a xenogenic species, for example the human adaptative system, *in vivo.*

The invention relates also to a method for analysing the development of the immune system of a xenogenic species *in vivo,* for example the human immune system *in vivo,* comprising the step of:
- transplanting xenogenic hematopoietic progenitor cells from the same species as the MHC class I and/or class II transgene(s), for example human hematopoietic progenitor cells, into a transgenic mouse as defined above, by any appropriate means, and
- assaying for the presence of functional T, B or dendritic cells of the xenogenic species, for example human, in the transplanted mouse, by any appropriate means.

The presence of functional human T-, B- and dendritic (DC)- cells is assayed by any technique well-known in the art.

Several weeks after transplantation (from 4 weeks to about 12 weeks), bone marrow, spleen, thymus and peripheral blood may be analysed by flow cytometry and/or immunohistochemistry, based on expression of appropriate markers. The level of engraftment is determined by measuring the human leukocytes (CD45⁺) cell number and the proportion of T cells (CD45⁺, CD3⁺), B cells (CD45⁺, CD19⁺) and dendritic cells (CD45⁺, CD11⁺ and CD45⁺, CD11⁻CD123⁺) in the different lymphoid organs.

The presence of functional human T cells may be assayed measuring the proliferative capacity of T cells, for example to an alloantigen (Mixed Lymphocyte reaction) or their capacity to produce cytokines (ELISPOT, intracellular flow cytometry). The diversity of the T cell repertoire may be assayed by immunoscope analyses.

The presence of functional human B cells may be assayed by measuring, either the total human immunoglobulins in mice sera, the hIgG and the IgM by ELISA or the immunoglobulin secreting cells, by ELISPOT.

The presence of functional DC may be assayed *ex vivo* by measuring their capacity to induce the proliferation of allogenic T cells (CD11⁻ cells) or their capacity to produce IFN-α after stimulation (CD11⁺ cells).

The invention relates also to a method for analysing the immune response of a xenogenic species to an antigen *in vivo,* for example the human immune response to an antigen *in vivo,* comprising the steps of:
- transplanting xenogenic hematopoietic progenitor cells from the same species as the MHC class I and/or class II transgene(s), for example human hematopoietic progenitor cells, into a transgenic mouse as defined above, by any appropriate means,
- bringing the transplanted cells into contact with an antigen of interest, and,
- assaying for the presence of a humoral response, a T-helper cell response or a T-cytotoxic cell response to the antigen, in the mouse, by any appropriate means.

The antigen is preferably injected after a time sufficient to allow the reconstitution of a functional immune system. Alternatively, it may be interesting to assess the effect of antigen on immune development, for example T cell maturation.

The antigen may be natural, recombinant or synthetic. The antigen can comprise a polypeptide sequence or a polynucleotide sequence, which can comprise RNA, DNA, or both. In one embodiment, the antigen comprises at least one polynucleotide sequence operationally encoding one or more antigenic polypeptides. Used in this context, the word "comprises" intends that at least one antigenic polypeptide is provided by the transcription and/or translation apparatus of a host cell acting upon an exogenous polynucleotide that encodes at least one antigenic polypeptide. Antigens of the invention can be any antigenic molecule. Antigenic molecules include: proteins, lipoproteins, and glycoproteins, including viral, bacterial, parasitic, animal, and fungal proteins such as albumins, tetanus toxoid, diphtheria toxoid, pertussis toxoid, bacterial outer membrane proteins (including meningococcal outer membrane protein), RSV-F protein, malarial derived peptide, B-lactoglobulin B, aprotinin, ovalbumin, lysozyme, and tumor associated antigens such-as carcinoembryonic antigen (CEA), CA 15-3, CA 125, CA 19-9, prostrate specific antigen (PSA); carbohydrates, including naturally-occurring and synthetic polysaccharides and other polymers such as ficoll, dextran, carboxymethyl cellulose, agarose, polyacrylamide and other acrylic resins, poly (lactide-co-glycolide), polyvinyl alcohol, partially hydrolyzed polyvinyl acetate, polyvinylpryrolidine, Group B Steptococcal and Pneumococcal capsular polysaccharides (including type III), *Pseudomonas aeruginosa* mucoexopolysaccharide, and capsular polysaccharides (including fisher type I), and *Haemophilus influenzae* polysaccharides (including PRP); haptens, and other moieties comprising low molecular weight molecules, such as TNP, saccharides, oligosaccharides, polysaccharides, peptides, toxins, drugs, chemicals, and allergens; and haptens and antigens derived from bacteria, fungi, viruses, parasites and prions. Bacteria include for example: C. *diphtheriae, B.pertussis, C. tetani, H. influenzae, S. pneumoniae, E. Coli,* Klebsiella, *S*. *aureus, S. epidermidis, N. meningiditis, B. anthracis,* Listeria, *Chlamydia trachomatis* and *pneumoniae,* Rickettsiae, Group A Streptococcus, Group B Streptococcus, *Pseudomonas aeruginosa,* Salmonella, Shigella, Mycobacteria *(Mycobacterium tuberculosis)* and Mycoplasma. Viruses include for example: Polio, Mumps, Measles, Rubella, Rabies, Ebola, Hepatitis A, B, C, D and E, Varicella Zoster, Herpes simplex types 1 and 2, Parainfluenzae, types 1, 2 and 3 viruses, Human Immunodeficiency Virus I and II, , RSV, CMV, EBV, Rhinovirus, Influenzae virus A and B, Adenovirus, Coronavirus, Rotavirus and Enterovirus. Fungi include for example: *Candida sp. (Candida albicans).* Parasites include for example: Plasmodium *(Plasmodium falciparum), Pneumocystis carinii,* Leishmania, and Toxoplasma.

The presence of an immune response to the antigen is assayed by any technique well-known in the art.

The presence of a humoral response to the antigen is assayed by measuring, either the titer of antigen-specific antibodies in mice sera, by ELISA, or the number of antibody secreting cells, by ELISPOT.

The presence of a T-helper cell response to the antigen is assayed by a T cell proliferation assay or cytokine production assays (ELISPOT or intracellular flow cytometry).

The presence of a T-cytotoxic cell response to the antigen is assayed by a CTL assay *in vitro* or *in vivo* (Barber DL et al., J Immunol, 2003, 171:27-31).

The method of the invention may be used for mapping antigens, for screening new antigens, as well as for evaluating the immunogenicity of different antigen preparations for use as human vaccine.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); DNA Cloning, Volumes I and II (D. N. Glover ed., 1985); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-inchief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In addition to the preceding features, the invention further comprises other features which will emerge from the description which follows, which refers to examples illustrating the transgenic mice and their uses according to the invention, as well as to the appended drawings in which:

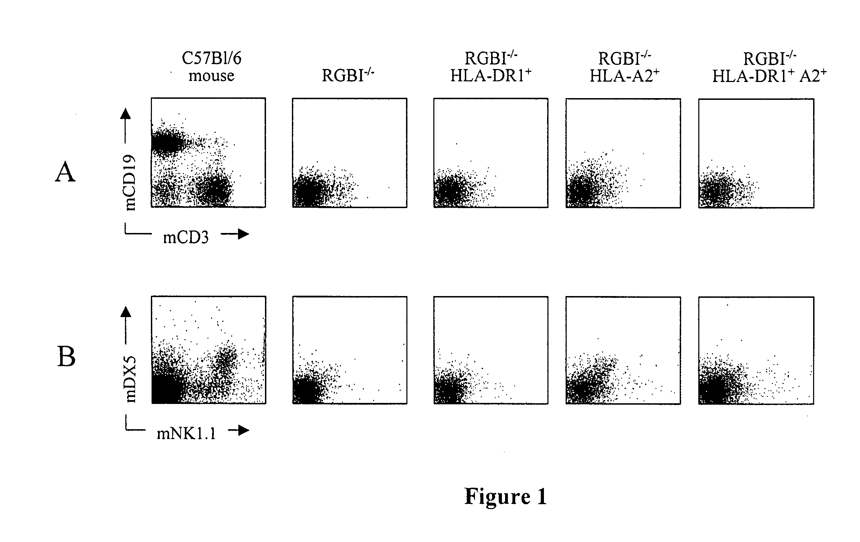

- figure 1 illustrates the absence of T, B and NK T cells in the transgenic mice. Splenocytes from C57B1/6 mice, Rag^{-/-} γc^{-/-}β2m^{-/-} IAβ^{b-/-} (referred as RGBI^{-/-}), Rag^{-/-} γc^{-/-}β2m^{-/-} IAβ^{b-/-} HLA-DR1⁺, Rag^{-/-} γc^{-/-}β2m^{-/-} IAβ^{b-/-} HLA-A2⁺, Rag^{-/-} γc^{-/-}β2m^{-/-} IAβ^{b-/-} HLA-DR1⁺ HLA-A2⁺ mice were stained for murine CD19 and murine CD3 (A) and murine NK1.1 and murine DX5 (B). The panels are representative of three mice of each group and two human donors.

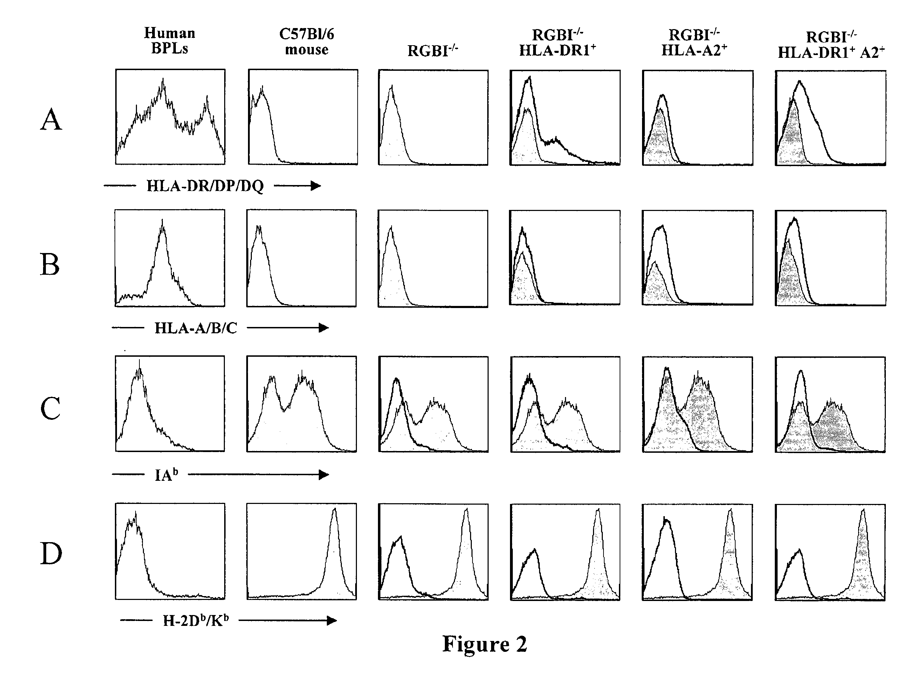

- figure 2 illustrates the analysis of MHC molecule expression in the transgenic mice. Human PBLs and splenocytes from C57B1/6 mice, Rag^{-/-} γc^{-/-}β2m^{-/-} IAβ^{b-/-} (referred as RGBI^{-/-}), Rag^{-/-} γc^{-/-}β2m^{-/-} IAβ^{b-/-} HLA-DR1⁺, Rag^{-/-} γc^{-/-}β2^{m-/-} IAβ^{b-/-} HLA-A2⁺, Rag^{-/-}γc^{-/-}β₂m^{-/-} IAβ^{b-/-} HLA-DR1⁺ HLA-A2⁺ mice were stained for HLA-DR/DP/DQ (A), HLA-A/B/C (B), IA^{b} (C), H-2D^{b}/K^{b} (D).

For Rag^{-/-}γc^{-/-}β2m^{-/-} IAβ^{b-/-} HLA-DR1⁺, Rag^{-/-}-γc^{-/-}β2m^{-/-} IAβb^{-/-} HLA-A2⁺, Rag^{-/-} γc^{-/-}β2m^{-/-} IAβ^{b-/-} HLA-DR1⁺ HLA-A2⁺ mice, HLA-DR/DP/DQ (A), and HLA-A/B/C (B) expression profile (opened histograms) are shown together with that obtained for Rag^{-/-}γc^{-/-}β2m^{-/-} IAβ^{b-/-} control mice (filled histograms).

For Rag^{-/-}γc^{-/-}β2m^{-/-} IAβb^{-/-}, Rag^{-/-}γc^{-/-}β2m^{-/-} IAβ^{b-/-} HLA-DR1⁺, Rag^{-/-} γc^{-/-}β2m^{-/-} IAβ^{β-/-} HLA-A2⁺, Rag^{-/-} γc^{-/-}β2m^{-/-} IAβ^{b-/-} HLA-DR1⁺ HLA-A2⁺ mice, IAβ^{b} (C) and H-2D^{b}/K^{b} (D). Expression profiles (opened histograms) are shown together with that obtained for C57B1/6 control mice (filled histograms).

The panels are representative of three mice of each group and two human donors.

### Example 1: Transgenic mice production and characterization

### 1) Material and methods

### a) Mice

The transgenic mice were produced by crossing of different mice strains:
- Rag^{2-/-}, γ_{c}^{-/-} (129 background), obtained by crossing Rag2^{-/-} mice (Takeda et al., Immunity, 1996, 5, 217-228) with γ_{c}^{-/-} mice (DiSanto et al., P.N.A.S., 1995, 92, 377-381).
- β₂m^{-/-} (C57B1/6 background), described in Zijlstra et al., Nature, 1990, 344, 742-746.
- I-Aβ^{b-/-} (C57B1/6 background), described in Takeda et al., Immunity, 1996, 5, 217-228.
- HLA-DR1 transgenic mice (DR1⁺ , FVB background), expressing the DR1 molecule encoded by the HLA-DRA*0101 and HLA-DRB1*0101 gene, described in Altmann et al., J. Exp. Med., 1995, 181, 867-875.
- HLA-A2.1 transgenic mice, expressing a chimeric monochain (α1-α2 domains of HLA-A2.1 (encoded by HLA-A*0201 gene), α3 to cytoplasmic domains of H-2 D^{b}, linked at its N-terminus to the C terminus of human β2m by a 15 amino-acid peptide linker), described in Pascolo et al., J. Exp. Med., 1997, 185, 2043-2051.

More precisely, the Rag2^{-/-}, γ_{c}^{-/-} mice (129 background) were crossed with I-Aβ^{b-/-} (C57B1/6 background) and the F1 progeny were crossed successively with β₂m^{-/-} (C57B1/6 background), HLA-DR1 transgenic mice (DR1⁺, FVB background) and HLA-A2.1 transgenic mice. I-Aα^{b+} (essential for murine MHC class II ^{-/-} phenotype) and C5 ^{-/-} progenies (both 129 and FVB strains are constitutively C5^{-/-}) were selected from each crossing.

### b) Genotyping

The genotype of each progeny was determined by PCR on tail-DNA using standard protocols and the following primers for the different genes:

**Table I: Primers used for genotyping**

| **Gene** | **Primer sequences (SEQ ID NO : 1 to 22)** |
|---|---|
| Rag2 | RagA : GGG AGG ACA CTC ACTTGC CAG TA |
| | RagB : AGT CAG GAG TCT CCA TCT CAC TGA |
| | neo :CGG CCG GAG AAC CTG CGT GCAA |
| γc | GCF1 : AGC TCC AAG GTC CTCATG TCC AGT |
| | GCF2 : GTG TAC TGT TGG TTGGAA CGG TGA G |
| | GCR : GGG GAG GTT AGC GTCACT TAG GAC |
| I-Aα^{b} | Ea5' : AGT CTT CCC AGC CTT CAC ACT CAG AGG TAC |
| | EA3' : CAT AGC CCC AAA TGT CTG ACC TCT GGA GAG |
| I-Aβ^{b} | Iaß^{b}f : CCG TCC GCA GGG CATTTC GTG TA |
| | Iaß^{b}r : AGG ATC TCC GGC TGGCTG TTC |
| β2m | ß2M fwd : AAT GGG AAG CCG AACATA CTG AAC |
| | ß2M rvs : GTC ATG CTT AAC TCTGCA GGC GTA |
| | neo : CTT AAT ATG CGA AGTGGA CCT GGG |
| HLA-DR1 | DR1f :TTC TTC AAC GGG ACG GAG CGC GTG |
| | DR1r : CTG CAC TGT GAA GCTCTC ACC AAC |
| HLA-A2 | B2hAS1332 : GGA TGA CGT GAG TAAACC TGA ATC TTT GGAGTA CGC |
| | PROMA2S964 : CAT TGA GAC AGA GCGCTT GGC ACA GAA GCAG |
| C5 | C5-20 TGG CAT TTC AGA CAATGG TAG |
| | C5-21 : GTG TAT CAG CAA CACATA TAC |
| | 945 : AAG CAC TAG CAT CTCAAA CAA |
| | C5pos CAA CAA CTG GAA CTGCAT AC |
| | C5neg : ACA ACA ACT GGA ACTGCA CT |

### c) Flow cytometry analysis

Human PBLs, splenocytes of C57B1/6, Rag2^{-/-}, γ_{c}^{-/-}, β₂m^{-/-}, I-Aβ^{b -/-}, HLA-A2^{+/+}, HLA-DR1^{+/+}, Rag2^{-/-}, γ_{c}^{-/-}, β₂m^{-/-}, I-Aβ^{b -/-}, HLA-A2^{+/+}, Rag2^{-/-}, γ_{c}^{-/-}, β₂m^{-/-}, I-Aβ^{b -/-}, HLA-DR1^{+/+}, and Rag2^{-/-}, γ_{c}^{-/-}, β₂m^{-/-}, I-Aβ^{b-/-} mice were dilacerated and incubated with anti-FcR antibody. The cells were then stained in PBS containing 0.05 % azide, 2 % FCS and anti-HLA-DR/DP/DQ, anti-HLA-A/B/C, anti-H-2 IA^{b}, anti-H-2D^{b} + anti-K^{b}, anti-mouse CD 19, anti-mouse CD3, anti-mouse NK 1.1 monoclonal antibodies (mAbs) conjugated with appropriate fluorochrome. After staining, cells were washed twice and resuspended in the same buffer. Labelling was analyzed on a LSR flow cytometer with CellQuest software.

### 2) Results

Four mice strains were obtained :
- Rag2^{-/-}, γ_{c}^{-/-}, β2m^{-/-}, I-Aβ^{b-/-}, HLA-A2^{+/+}, HLA-DR1^{+/+},
⁻ Rag2^{-/-}, γ_{c}^{-/-}, β₂m^{-/-}, I-Aβ^{b-/-}, HLA-A2^{+/+},
-Rag2^{-/-}, γ_{c}^{-/-}, β₂m^{-/-}, I-Aβ^{b-/-}, HLA-DR1^{+/+}, and
- Rag2^{-/-}, γ_{c}^{-/-}, β₂m^{-/-}, I-Aβ^{b-/}-.

The absence of murine T, B (Figure 1A) and NK cells (Figure 1B) in the four types of mice was evaluated by fluorescence-activated cell sorting. Cell surface expression of the HLA-A2.1, H-2 K^{b}/D^{b}, HLA-DR1 and H-2 IA^{b} molecules was evaluated by flow cytometry. Cell surface expression of endogenous H-2 class I and class II molecules was absent in the four mice (Figure 2C and 2D). A similar level of HLA-A2.1 expression (Figure 2B) was observed in HLA A2.1 /HLA-DR1 transgenic H-2 class I-/class II-KO immunodeficient mice and HLA A2.1 transgenic H-2 class I-/class II-KO immunodeficient mice, while HLA-A2.1 was absent in HLA-DR1 transgenic H-2 class I-/class II-KO mice and H-2 class I-/class II-KO immunodeficient mice. A similar level of HLADR1 expression (Figure 2A) was observed in HLA A2. 1-/HLA-DR1-transgenic H-2 class I-/class II-KO immunodeficient mice and HLA-DR1-transgenic H-2 class I-/class II-KO immunodeficient mice, whereas no expression was detected in HLA A2.1-transgenic H-2 class I-/class II-KO immunodeficient mice and H-2 class I-/class II-KO immunodeficient mice. HLA-DR1 and -A2 molecules are, however, lower in transgenic mice than in human PBLs.

### Example 2: Human/mouse chimera production and characterization

CD34⁺ cells were derived from human embryonic stem cells according to the procedure described in Vodyanik et al., Blood, 2005, 105, 617-626. Briefly, ES cells were co-cultured for 6-9 days on 4 day-old confluent OP-9 murine bone marrow stromal cell line. CD34⁺ percentage was determined by flow cytometry using an anti-human CD34 PE conjugated mAbs (BECTON DICKINSON). Single cell suspension was then prepared by treatment by collagenase IV (INVITROGEN) and CD34⁺ cells were magnetically sorted using Direct CD34 progenitor Cell Isolation Kit (MILTENYI BIOTECH) as recommended by the manufacturer, and processed on an Automacs machine (MILTENYI BIOTECH).

At day of birth, newborn mice were irradiated in a 4 hour interval with 2x2 Gy from a Cesium 137 source at 4 Gy/min, a dose that was titrated to be sub lethal. At 4-12 hours post irradiation, mice were transplanted with human CD34⁺ cells in 20 µl PBS into the liver (i.h.) using a 30-gauge needle.

Three months after transplantation, mice were bled from tail vein, to obtain peripheral blood cells and plasma. The mice where then sacrificed and single cell suspensions from organs (bone marrow, thymus, spleen, lymph nodes) were prepared.

The presence of T (CD3⁺ CD4⁺ and CD8⁺), B (CD19), NK (CD 16), DC (CD 11c⁺ CD 123^{+/-}) and macrophages (CD 11b⁺) was assessed by flow cytometry.

The diversity of T repertoire was analyzed using the immunoscope method. The functionality of T cells was analyzed in a MLR assay for proliferation, by using the CFSE labeling technique and in an intracellular IL-2 and IFN-γ cytokine flow cytometry assay. The functionality of B cells was assessed by testing the sera for the level of total IgGs and IgMs, by ELISA.

### Example 3 : Use of the human/mouse chimera to study the immune response to a pathogen

The human/mouse chimera are used as a model for HIV infection and in particular to study the role of CD8 T cells in the control and/or pathogenesis of this infection.

Human/mouse chimera already reconstituted by human immune system, as described in example 2, were injected intraperitoneally with either HIV virus alone (viral strains or primary isolates) or infected PHA-blastic allogenic T cells. Blood samples were taken at different time points post-infection to monitor both the percentage of human CD4 and CD8 T cells by flow cytometry, and the plasmatic viral load (proviral DNA and viral RNA).

The anti-viral T cell responses in infected chimera was evaluated and compared with the response obtained by T cells from non-infected chimera, in the following assays:
- the expansion of anti-viral T cells from chimera spleen and lymph nodes was measured by flow cytometry using tetramers of different HLA-DR1- or HLA-A2-restricted HIV peptide complexes,
- the production of γIFN, αTNF and IL-2 was assessed after in *vitro* restimulation of spleen and lymph nodes T cells by different HLA-DR1- or HLA-A2-restricted HIV peptides, using ELISPOT or flow cytometry (intracellular staining), and
- the anti-viral cytotoxicity was measured by *in vitro* ⁵¹Cr release assay and *in vivo* injection of CFSE-labeled syngenic B cell targets loaded or non-loaded with different HLA-A2-restricted HIV peptides.

The role of CD8 T cells during HIV infection was evaluated by CD8 T cells depletion and infusion:
- infected chimera were depleted of CD8 T cells using injection of anti-human CD8 antibodies. The depletion was checked by flow cytometry from blood samples. The percentage of CD4 T cells and the splenic and lymph node viral loads were then compared between CD8-depleted and CD4-replete HIV-infected chimera, and
- syngenic CD8 T cells from HIV-infected chimera were adoptively transferred into CD8 T cell-depleted HIV infected chimera. The percentage of CD4 T cells and the splenic and lymph node viral loads were then compared between CD8-injected and CD8-non-injected HIV-infected chimera.

The role of CD8 T cells on HIV susceptibility was evaluated in CD8-depleted and CD8-replete chimera, injected by HIV. Productive infection was assessed and compared between CD8-depleted and CD8-replete chimera at different time points post-injection. Therefore, blood, thymus, spleen and lymph node cells were tested for the presence of virus by RT-PCR (viral load) and pro-virus by PCR (proviral load), before and after PHA-stimulation.

## Claims

1. A transgenic mouse, **characterized in that** it has a phenotype comprising:
a) a deficiency for murine T lymphocytes, B lymphocytes and NK cells,
b) a deficiency for murine MHC class I and MHC class II molecules, and
c) a functional xenogenic MHC class I transgene and/or a functional xenogenic MHC class II transgene.

2. The transgenic mouse according to claim 1, wherein the deficiency in a) is associated with a deficient Rag2 gene and a deficient common receptor γ chain gene.

3. The transgenic mouse according to claim 1 or claim 2, wherein the deficiency in murine MHC class I molecules is associated with a deficient β2-microglobulin gene.

4. The transgenic mouse according to anyone of claims 1 to 3, wherein the deficiency in murine MHC class II molecules is associated with a deficient H-2^{b}-Aβ gene.

5. The transgenic mouse according to anyone of claims 1 to 4, wherein the xenogenic MHC class I and/or class II transgenes are human HLA class I and/or HLA class II transgenes.

6. The transgenic mouse according to claim 5, wherein the HLA class I transgene is an HLA-A2 transgene and the HLA class II transgene is an HLA-DR1 transgene.

7. The transgenic mouse according to claim 6, having a genotype selected from the group consisting of :
- Rag2^{-/-}, γ_{c}^{-/-}, β₂m^{-/-}, I-Aβ^{b-/-}, HLA-A2^{+/+}, HLA-DR1^{+/+},
- Rag2^{-/-}, γ_{c}^{-/-}, β₂m^{-/-}, I-Aβ^{b-/-}, HLA-A2^{+/+}, and
⁻Rag2^{-/-}, γ_{c}^{-/-}, β₂m^{-/-}, I-Aβ^{b-/-}, HLA-DR1^{+/+}.

8. The transgenic mouse according to anyone of claims 1 to 7, **characterized in that** it further comprises a deficiency for the C5 protein of complement.

9. A method for producing a chimeric mouse having a functional xenogenic immune system, **characterized in that** it comprises the step of: transplanting xenogenic hematopoietic progenitor cells from the same species as the MHC class I and/or class II transgene, into a transgenic mouse according to anyone of claims 1 to 8, by any appropriate mean.

10. A method for analysing the development of the immune system of a xenogenic species *in vivo,* **characterized in that** it comprises the steps of:
- transplanting xenogenic hematopoietic progenitor cells from the same species as the MHC class I and/or class II transgene, into a transgenic mouse according to anyone of claims 1 to 8, by any appropriate means, and
- assaying for the presence of functional T, B or dendritic cells of the xenogenic species, in the transplanted mouse, by any appropriate means.

11. A method for analysing the immune response of a xenogenic species to an antigen *in vivo,* **characterized in that** it comprises the steps of:
- transplanting xenogenic hematopoietic progenitor cells from the same species as the MHC class I and/or class II transgene, into a transgenic mouse according to anyone of claims 1 to 8, by any appropriate means,
- bringing the transplanted cells into contact with an antigen of interest, and,
- assaying for the presence of a humoral response, a T-helper cell response or a T-cytotoxic cell response to the antigen, in the mouse, by any appropriate means.

12. The method according to anyone of claims 8 to 10, wherein the xenogenic hematopoietic progenitor cells are human hematopoietic progenitor cells.

13. The method according to claim 12, wherein the human hematopoietic progenitor cells are from donors of different HLA haplotypes.

14. The method according to claim 13, wherein said haplotypes are reflective of the genetic variability of the human population.

15. A transgenic mouse useful for the production of a transgenic mouse according to anyone of claims 1 to 8, **characterized in that** it has a phenotype comprising :
a) a deficiency for murine T lymphocytes, B lymphocytes and NK cells, and
b) a deficiency for murine MHC class I and MHC class II molecules.

16. The transgenic mouse according to claim 15, **characterized in that** it has a Rag2^{-/-}, γc^{-/-}, β₂m^{-/-}, I-Aβ^{b-/-} genotype.

17. An isolated cell, **characterized in that** it is a cell from a transgenic mouse according to anyone of claims 1 to 8, 15 and 16.


SEQUENCE LISTING

<110>  INSTITUT PASTEUR

<120>  IMMUNODEFICIENT MICE TRANSGENIC FOR HLA CLASS I AND HLA CLASS II
       MOLECULES AND THEIR USES

<130>  226ep129

<160>  22

<170>  PatentIn version 3.3

<210>  1
<211>  23
<212>  DNA
<213>  ARTIFICIAL SEQUENCE

<220>
<223>  PRIMER

<400>  1
gggaggacac tcacttgcca gta                                              23


<210>  2
<211>  24
<212>  DNA
<213>  ARTIFICIAL SEQUENCE

<220>
<223>  PRIMER

<400>  2
agtcaggagt ctccatctca ctga                                             24


<210>  3
<211>  22
<212>  DNA
<213>  ARTIFICIAL SEQUENCE

<220>
<223>  PRIMER

<400>  3
cggccggaga acctgcgtgc aa                                               22


<210>  4
<211>  24
<212>  DNA
<213>  ARTIFICIAL SEQUENCE

<220>
<223>  PRIMER

<400>  4
agctccaagg tcctcatgtc cagt                                             24


<210>  5
<211>  25
<212>  DNA
<213>  ARTIFICIAL SEQUENCE

<220>
<223>  PRIMER

<400>  5
gtgtactgtt ggttggaacg gtgag                                              25


<210>  6
<211>  24
<212>  DNA
<213>  ARTIFICIAL SEQUENCE

<220>
<223>  PRIMER

<400>  6
ggggaggtta gcgtcactta ggac                                               24


<210>  7
<211>  30
<212>  DNA
<213>  ARTIFICIAL SEQUENCE

<220>
<223>  PRIMER

<400>  7
agtcttccca gccttcacac tcagaggtac                                         30


<210>  8
<211>  30
<212>  DNA
<213>  ARTIFICIAL SEQUENCE

<220>
<223>  PRIMER

<400>  8
catagcccca aatgtctgac ctctggagag                                         30


<210>  9
<211>  23
<212>  DNA
<213>  ARTIFICIAL SEQUENCE

<220>
<223>  PRIMER

<400>  9
ccgtccgcag ggcatttcgt gta                                                23


<210>  10
<211>  21
<212>  DNA
<213>  ARTIFICIAL SEQUENCE

<220>
<223>  PRIMER

<400>  10
aggatctccg gctggctgtt c                                                  21


<210>  11
<211>  24
<212>  DNA

<213>  ARTIFICIAL SEQUENCE

<220>
<223>  PRIMER

<400>  11
aatgggaagc cgaacatact gaac                                              24


<210>  12
<211>  24
<212>  DNA
<213>  ARTIFICIAL SEQUENCE

<220>
<223>  PRIMER

<400>  12
gtcatgctta actctgcagg cgta                                              24


<210>  13
<211>  24
<212>  DNA
<213>  ARTIFICIAL SEQUENCE

<220>
<223>  PRIMER

<400>  13
cttaatatgc gaagtggacc tggg                                              24


<210>  14
<211>  24
<212>  DNA
<213>  ARTIFICIAL SEQUENCE

<220>
<223>  PRIMER

<400>  14
ttcttcaacg ggacggagcg cgtg                                              24


<210>  15
<211>  24
<212>  DNA
<213>  ARTIFICIAL SEQUENCE

<220>
<223>  PRIMER

<400>  15
ctgcactgtg aagctctcac caac                                              24


<210>  16
<211>  36
<212>  DNA
<213>  ARTIFICIAL SEQUENCE

<220>
<223>  PRIMER

<400>  16
ggatgacgtg agtaaacctg aatctttgga gtacgc                                 36

<210>  17
<211>  31
<212>  DNA
<213>  ARTIFICIAL SEQUENCE

<220>
<223>  PRIMER

<400>  17
cattgagaca gagcgcttgg cacagaagca g                                    31


<210>  18
<211>  21
<212>  DNA
<213>  ARTIFICIAL SEQUENCE

<220>
<223>  PRIMER

<400>  18
tggcatttca gacaatggta g                                               21


<210>  19
<211>  21
<212>  DNA
<213>  ARTIFICIAL SEQUENCE

<220>
<223>  PRIMER

<400>  19
gtgtatcagc aacacatata c                                               21


<210>  20
<211>  21
<212>  DNA
<213>  ARTIFICIAL SEQUENCE

<220>
<223>  PRIMER

<400>  20
aagcactagc atctcaaaca a                                               21


<210>  21
<211>  20
<212>  DNA
<213>  ARTIFICIAL SEQUENCE

<220>
<223>  PRIMER

<400>  21
caacaactgg aactgcatac                                                 20


<210>  22
<211>  20
<212>  DNA
<213>  ARTIFICIAL SEQUENCE

<220>
<223>  PRIMER

<400> 22
acaacaactg gaactgcact 20